# EUROPEAN PATENT APPLICATION

(11) **EP 4 112 023 A1**
(43) Date of publication of application: **04.01.2023**
(21) Application number: 21759807.7
(22) Date of filing: 09.02.2021
(51) Int. Cl.: A61F 13/15, A61F 13/56

(54) **PACKAGING SHEET AND PACKAGED ABSORBENT ARTICLE**

(30) Priority: 27.02.2020 JP 2020031876
(71) Applicant: DAIO PAPER CORPORATION, Shikokuchuo-shi Ehime 799-0492 (JP)
(72) Inventor: YOSHIBA, Megumi, Sakura-shi, Tochigi 329-1411 (JP)
(74) Representative: Epping - Hermann - Fischer
(86) International application number: PCT/JP2021/004833
(87) International publication number: WO 2021/172006

(57) **Abstract**

A wrapping sheet for wrapping an absorbent article is provided. The wrapping sheet includes a first region and a second region. The first region includes one end in a first direction of the wrapping sheet and the second region includes an opposite end in the first direction. The wrapping sheet is configured to wrap the absorbent article by causing the first region to overlap with the outer surface of the second region, and a sealing tape is attached so as to extend from a first portion on the outer surface of the first region to a second portion on the outer surface of the second region. The outer surface of the second portion is subjected to treatment so as to reduce a bonding force between the second portion and the sealing tape as compared to a bonding force between the first portion and the sealing tape.

## Description

### TECHNICAL FIELD

The present invention relates to a wrapping sheet and a wrapped absorbent article.

### BACKGROUND ART

Generally, absorbent articles such as sanitary napkins, panty liners, and incontinence pads are provided in a wrapped form in a wrapping sheet for hygiene and portability purposes.

As such a wrapped absorbent article (a wrapped absorbent article or an individual package of an absorbent article), Patent Document 1 describes an individual package in which an overlapping portion is formed by folding a wrapping sheet such that the front edge portion is located on the outermost side of the wrapping sheet, and the side edge portions of the wrapping sheet are removably sealed. In the individual package, a base end of a tape is fixed to the outer surface of the overlapping portion of the wrapping sheet, and a free end of the tape extends across the front edge portion of the wrapping sheet and is removably attached to the outer surface of a non-overlapping portion of the wrapping sheet.

Patent Document 1 describes that the surface of the wrapping sheet, to which the base end of the tape is attached, is smoothed by a smoothing process, and this smoothing process allows the base end of the tape to be securely fixed to the wrapping sheet, which is a non-woven fabric.

### RELATED-ART DOCUMENTS

### PATENT DOCUMENTS

Patent Document 1: Japanese Laid-open Patent Publication No. 2003-175990

### SUMMARY OF THE INVENTION

### PROBLEM TO BE SOLVED BY THE INVENTION

However, in the individual package described in Patent Document 1, no specific design considerations are made for the outer surface of the wrapping sheet to which the free end of the tape is attached. Therefore, if a tape having high adhesiveness is used, it may be difficult to readily peel the free end of the tape from the wrapping sheet when opening the individual package. In such a case, if the free end of the tape is forcibly peeled with a large force, the wrapping sheet may be torn.

Conversely, if a tape having low adhesiveness is used in order to facilitate ease of peeling of the tape, the base end of the tape may be easily peeled as well. Therefore, there may be a possibility that the tape may be removed before the individual package is opened, and thus, the tape may fail to properly function.

One aspect of the present invention is to provide a wrapped absorbent article in which a tape is readily peeled when the wrapped absorbent article is opened, while maintaining the original function of the tape.

### MEANS TO SOLVE THE PROBLEM

According to one aspect of the present invention, a wrapping sheet for wrapping an absorbent article is provided. The wrapping sheet includes a first region and a second region. The first region includes one end in a first direction of the wrapping sheet and the second region includes an opposite end in the first direction. The wrapping sheet is configured to wrap the absorbent article by causing the first region to overlap with the outer surface of the second region, and a sealing tape is attached so as to extend from a first portion on the outer surface of the first region to a second portion on the outer surface of the second region. The outer surface of the second portion is subjected to treatment so as to reduce a bonding force between the second portion and the sealing tape as compared to a bonding force between the first portion and the sealing tape.

### EFFECTS OF THE INVENTION

According to one aspect of the present invention, a wrapped absorbent article in which, when the wrapped absorbent article is opened, a tape is readily peeled while maintaining the original function of the tape can be provided.

### BRIEF DESCRIPTION OF THE DRAWINGS

FIG. 1 is a plan view of a wrapped absorbent article that includes a wrapping sheet and an absorbent article according to an embodiment of the present invention;
FIG. 2 is a cross-sectional view of the wrapped absorbent article taken through I-I of FIG. 1;
FIG. 3 is a plan view of the partially unfolded wrapped absorbent article illustrated in FIG. 1;
FIG. 4 is a plan view of an unfolded state of the wrapping sheet, as viewed from the surface side of the wrapping sheet on which the absorbent article is placed, according to an embodiment of the present invention; and
FIG. 5 is a plan view of a wrapping sheet according to a modification.

### MODE FOR CARRYING OUT THE INVENTION

In the following, embodiments of the present invention will be described with reference to the accompanying drawings. In the drawings, unless otherwise noted, elements having the same or corresponding configurations are designated by the same reference numerals and the description thereof may be omitted. In order to facilitate understanding of the present invention, the drawings are schematically illustrated.

### (Wrapped Absorbent Article)

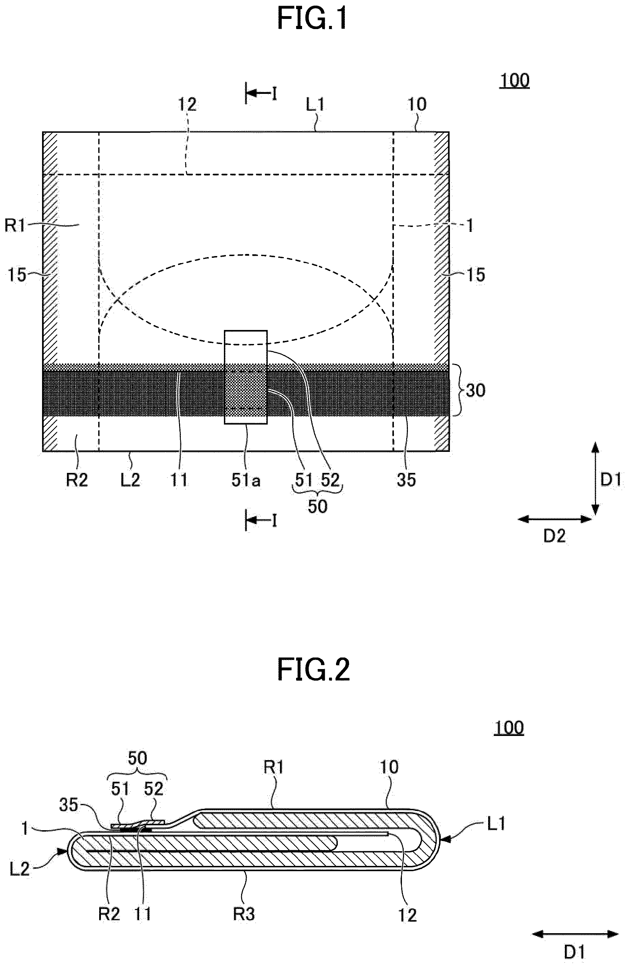

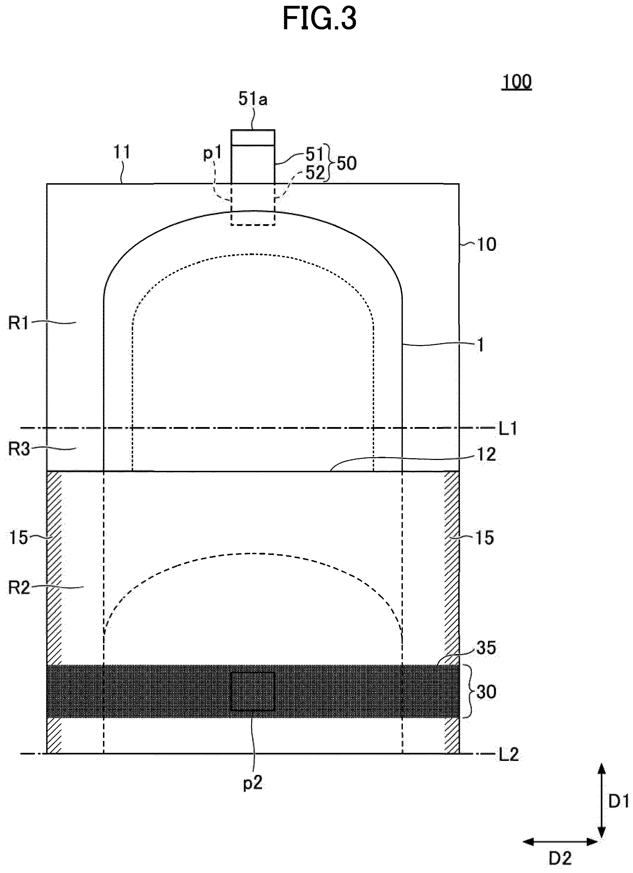

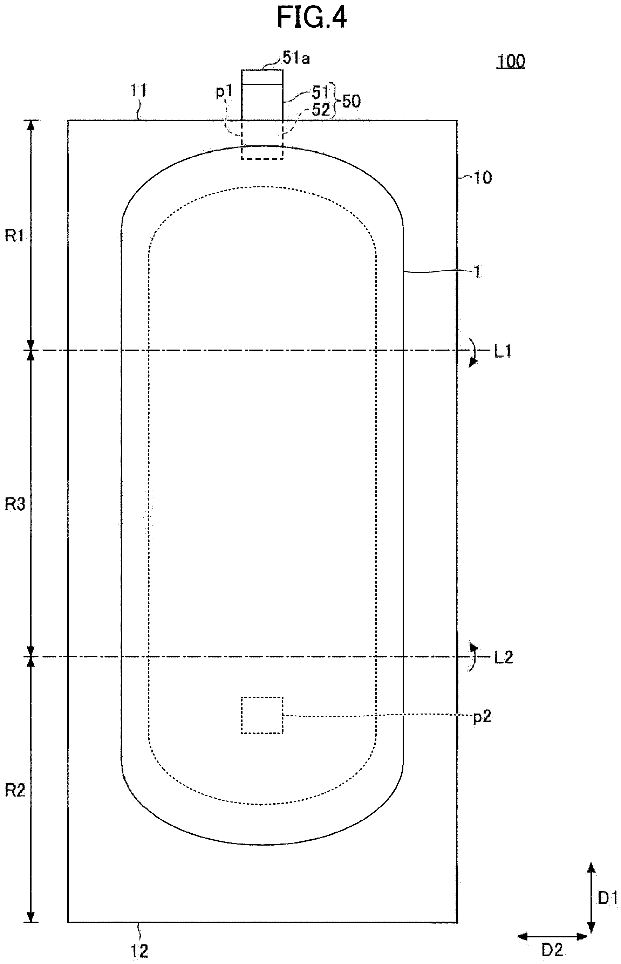

FIG. 1 is a plan view of a wrapped absorbent article 100 that includes a wrapping sheet 10 and an absorbent article 1 according to an embodiment of the present invention. FIG. 2 is a cross-sectional view of the wrapped absorbent article 100 taken through I-I of FIG. 1. FIG. 3 is a diagram illustrating the partially unfolded wrapped absorbent article 100 illustrated in FIG. 1. FIG. 4 is a plan view of an unfolded state of the wrapped absorbent article 100 as viewed from the outer surface side of the wrapping sheet 10.

As illustrated in FIG. 1 through FIG. 4, the wrapped absorbent article 100 includes the wrapping sheet 10 and the absorbent article 1 wrapped in the wrapping sheet 10. In other words, the wrapped absorbent article 100 is an individual package in which the single absorbent article 1 is enclosed by the single wrapping sheet 10. The wrapped absorbent article 100 can be obtained by placing the absorbent article 1 on the unfolded wrapping sheet 10, and folding the both ends in one direction of the wrapping sheet 10 so that the both ends overlap. That is, the wrapped absorbent article 100 can be obtained by tri-folding the wrapping sheet 10 together with the absorbent article 1.

More specifically, as illustrated in FIG. 3 and FIG. 4, the wrapping sheet 10 includes a first region R1 and a second region R2. The first region R1 includes one end 11 in the lengthwise direction D1 (first direction) of the wrapping sheet 10, and the second region R2 include an opposite end 12 in the lengthwise direction D1 of the wrapping sheet 10. The wrapping sheet 10 can wrap the absorbent article 1 by causing the first region R1 to overlap with the outer surface of the second region R2. At this time, the first region R1 is folded along a first folding line L1, which extends in the widthwise direction D2 (second direction) of the wrapping sheet 10. The widthwise direction D2 is perpendicular to the lengthwise direction D1. Further, the second region R2 is folded along a second folding line L2, which extends in the widthwise direction D2, before the first region R1 is folded. In the present embodiment, a region between the first region R1 and the second region R2 is referred to as a third region R3.

When the second region R2 is folded along the second folding line L2, the second region R2 may be folded such that the opposite end 12 in the lengthwise direction D1 reaches the first folding line L1, or the second region R2 may be folded such that the opposite end 12 is spaced apart from the first folding line L1 as illustrated in FIG. 1. Further, when the first region R1 is folded along the first folding line L1, preferably, the one end 11 in the lengthwise direction D1 of the wrapping sheet 10 does not extend beyond the second folding line L2 of the second region R2. A method of overlapping the two end regions of the wrapping sheet 10 as described above is easy and preferable. With this method, the absorbent article can be securely enclosed without being exposed to an external environment.

### (Absorbent Article)

The absorbent article 1 according to the embodiment of the present invention may be a sanitary napkin, a panty liner, an incontinence pad, or the like. In the present embodiment, the absorbent article 1 has an elongated shape in a plan view; however, the shape of the absorbent article 1 in a plan view is not limited to the elongated shape as illustrated in the drawings. For example, the absorbent article 1 may have any shape and may be wrapped in a wrapping sheet without being folded together with the wrapping sheet. For example, the absorbent article 1 may be a body fluid absorbent pad having a regular polygonal shape including a square shape, a circular shape, or the like. If the absorbent article 1 has an elongated shape, the entire length of the absorbent article 1 can be 140 mm to 420 mm, and the width of the absorbent article 1 can be 50 mm to 110 mm.

The absorbent article 1 may have a structure in which a liquid-impermeable back sheet, an absorbent body, and a liquid-permeable top sheet are laminated in this order. As the back sheet, a sheet material having at least a water shielding property, such as an olefin resin sheet formed of polyethylene or polypropylene, may be used. In addition, a laminated non-woven fabric formed by laminating a non-woven material onto a polyethylene sheet or the like, or a non-woven fabric laminated sheet that is substantially liquid-impermeable by interposing a waterproof film may be used. Further, a material having moisture permeability may be used.

As the top sheet, a porous or non-porous non-woven fabric, a porous plastic sheet, or the like may be preferably used. Examples of material fibers constituting the non-woven fabric include olefins such as polyethylene and polypropylene; synthetic fibers such as polyester and polyamide; regenerated fibers such as rayon and cuprammonium rayon; mixed fibers thereof; and natural fibers such as cotton. These fibers can be used alone or in combination with two or more kinds.

The material of the absorbent body is not particularly limited as long as the absorbent body can absorb and retain body fluids, and preferably includes cotton-like pulp and a water-absorptive polymer. Examples of the water-absorptive polymer include a superabsorbent polymer (SAP), a superabsorbent polymer fiber (SAF), and a combination thereof. Examples of the pulp include cellulose fibers such as dissolving pulp and chemical pulp made from wood, and synthetic cellulose fibers such as rayon and acetate.

The thickness of the absorbent body may be in the range of 0.5 mm to 25 mm, and preferably in the range of 1.5 mm to 6.5 mm. The absorbent body may have a structure in which a part of the absorbent body corresponding to a body fluid discharge region protrudes, or a part of the absorbent body facing a groove of the buttocks, located rearward of the body fluid discharge region, protrudes.

The absorbent body has a size and a shape so as not to extend beyond the top sheet and the back sheet. The outer edges of the back sheet and the top sheet, located forward of and rearward of the absorbent body, are bonded to each other with an adhesive such as a hotmelt adhesive or with an adhesive means such as a heat seal or an ultrasonic seal. Further, side non-woven fabrics, extending along the lengthwise direction, may be provided at both lateral sides of the absorbent body. As the side nonwoven fabrics, water-repellent nonwoven fabrics or hydrophilic non-woven fabrics may be used.

### (Wrapping Sheet)

The shape of the wrapping sheet 10 is not particularly limited. However, in an unfolded state as illustrated in FIG. 4, the wrapping sheet 10 preferably has an elongated shape, such as a rectangular shape or an elongated oval shape. Accordingly, the flat and elongated absorbent article 1 can be hygienically wrapped.

The size of the wrapping sheet 10 depends on the size and shape of the absorbent article 1 to be wrapped. For example, with the wrapping sheet 10 being unfolded, the length in the lengthwise direction D1 (which may be simply referred to as the "length") of the wrapping sheet 10 can be 100 mm to 450 mm, and the length in the widthwise direction D2 (which may be simply referred to as the "width"), which is orthogonal to the lengthwise direction D1, of the wrapping sheet 10 can be 70 mm to 250 mm.

Note that, if a relative long absorbent article is wrapped, a wrapping sheet 10 having a greater length can be used in accordance with the length of the absorbent article. In this case, the length of the second region R2 is set to be greater than the length of the third region R3. When the second region R2 is folded, first, a portion of the second region R2 is folded along a third folding line. The third folding line is located between the second folding line L2 and the opposite end 12 and extends in the widthwise direction D2. Then, the entire second region R2 is folded along the second folding line L2, and subsequently, the first region R1 is folded along the first folding line L1. In this manner, a quad-folded wrapped absorbent article can be obtained.

The material of the wrapping sheet 10 is not particularly limited, and may be a fiber-containing sheet such as paper or a non-woven fabric, or may be a resin film.

If paper is used as the material of the wrapping sheet 10, the environmental load at the time of disposal can be reduced and also a unique texture can be provided. As used herein, the "paper" refers to plant fibers or other fibers that are bonded together with an adhesive and formed in a flat sheet shape. In particular, the "paper" can refer to paper containing, as a main component, plant fibers (pulp), e.g., paper containing 50% or more of plant fibers and preferably containing 80% or more of plant fibers. Examples of types of pulp contained in paper include wood pulp, non-wood pulp, and waste paper pulp, which may be either mechanical pulp or chemical pulp. Further, additives may be added to paper. Specific examples of paper used for the wrapping sheet 10 include various types of paper, such as western paper, Japanese paper, coated paper, synthetic paper, and the like. Further, paper typically used for other purposes, such as newsprint paper, printing paper (including fine paper), writing paper, drawing paper, wrapping paper, thin paper, or other various types of paper can be used with or without being coated.

If paper is used as the material of the wrapping sheet 10, thin paper having a low basis weight can be used. If thin paper having a low basis weight is used as the material of the wrapping sheet 10, the wrapping sheet 10 can be readily folded when the absorbent article is wrapped, and the wrapped state can be readily maintained. The thin paper may be thin kraft paper, Indian paper, rice paper (used as cigarette paper or the like), glassine paper, tissue paper, toilet paper, filter paper, or the like. Further, the wrapping sheet 10 may be subjected to processing after papermaking. Examples of the above-described processing include creping, embossing, calendering, water-repellent coating (including forming a coating layer as will be described later), slitting, plying, printing, and the like. By performing creping and embossing, both strength and flexibility can be improved. In addition, if the wrapping sheet 10 is subjected to water-repellent coating, for example, a water-repellent agent containing a silicone-based resin, a paraffin-based resin, a fluoropolymer, or the like may be applied to at least one of the outer surface (which is exposed to the outside in a state in which the absorbent article 1 is wrapped) and the inner surface (which is opposite to the outer surface) of the wrapping sheet 10.

If paper is used as the material of the wrapping sheet 10, the basis weight of the wrapping sheet can be preferably less than or equal to 50 g/m², more preferably less than or equal to 40 g/m², and even more preferably less than or equal to 30 g/m², less than or equal to 20 g/m², less than or equal to 11 g/m², or less than or equal to 8 g/m². The lower limit of the basis weight of the wrapping sheet 10 is not particularly limited as long as the wrapping sheet 10 can properly function. The lower limit of the basis weight of the wrapping sheet 10 can be preferably greater than or equal to 5 g/m² and more preferably greater than or equal to 10 g/m². Further, if the wrapping sheet 10 is made of paper, the thickness of the wrapping sheet 10 can be preferably 40 µm to 200 µm, and more preferably 100 µm to 200 µm.

If a non-woven fabric is used as the material of the wrapping sheet 10, a texture and soft feel can be improved. A meltblown non-woven fabric or a spun-bonded non-woven fabric can be preferably used. Alternatively, a laminate of a plurality of layers of these non-woven fabrics can be used. Further, fibers constituting the non-woven fabric may be polyolefin such as polypropylene or polyethylene, or polyamide such as polyester or nylon.

If a resin film is used as the material of the wrapping sheet 10, polyolefin such as polypropylene or polyethylene, polyester, polyvinyl alcohol, or the like can be used as a resin. As a resin film, a stretched resin film is preferable. An air-impermeable film or an air-permeable film may be used. If a resin film is used, the outline of a print pattern can be made clear and good color development of a colorant can be provided, thus improving the design of the wrapping sheet 10.

The wrapping sheet 10 may be a single layer sheet in which one wrapping sheet 10 is composed of any of the above-described materials. The wrapping sheet 10 may be a laminated sheet formed by laminating a plurality of layers composed of different materials. For example, the wrapping sheet 10 may be a laminated sheet formed by laminating a resin film onto a paper sheet as described above.

As described in FIG. 1, the wrapped absorbent article 100 includes removable seal portions 15, 15 at both ends in the widthwise direction D2. The seal portions 15, 15 can prevent the absorbent article 1 from being exposed to the outside, and also prevent dust and the like from entering the inside of the wrapped absorbent article 100. The seal portions 15, 15 may be formed by heat sealing, ultrasonic sealing, or by using an adhesive.

### (Sealing Tape)

Further, after the absorbent article 1 is wrapped by the wrapping sheet 10, a sealing tape 50 is provided to the wrapped absorbent article 100 (FIG. 1 through FIG. 4). The sealing tape 50 is an adhesive tape having an adhesive layer on the wrapping sheet 10 side. The adhesive tape can be removably attached to the outer surface of the wrapping sheet 10 even after a certain period of time elapses.

The sealing tape 50 is attached to the wrapping sheet 10 so as to extend from the first region R1, which overlaps with the outer surface of the second region R2, to the second region R2. In other words, the sealing tape 50 is attached to the wrapping sheet 10 so as to extend across the one end 11 of the first region R1 in the lengthwise direction D1. By providing the sealing tape 50, at least a portion of the first region R1 can be secured to the second region R2 such that the first region R1 does not open. Accordingly, the sealing tape 50 can prevent dust from entering between the first region R1 and the second region R2 before the wrapped absorbent article 100 is opened. In addition, the sealing tape 50 can prevent the first region R1 from being peeled from the second region R2 due to a user's hand or an object accidentally entering between the first region R1 and the second region R2.

Typically, when opening the wrapped absorbent article 100, the user pulls a portion (that is, a free portion 51) of the sealing tape 50 attached to the second region R2 with one hand while holding a portion around the second folding line L2 of the wrapping sheet 10 with the other hand, and peels the sealing tape 50 from the second region R2. Then, when the sealing tape 50 is further pulled, the seal portions 15, 15 are unsealed, but a portion (that is, a fixed portion 52) of the sealing tape 50 attached to the first region R1 remains attached to the first region R1. Specifically, the first region R1 is lifted together with the sealing tape 50 and peeled from the second region R2 by continuously pulling the sealing tape 50, and as a result, the wrapped absorbent article 100 can be unfolded.

A portion of the first region R1 to which the sealing tape 50 is attached is referred to as a first portion p1. Further, a portion of the second region R2 to which the sealing tape 50 is attached is referred to as a second portion p2. In other words, in the wrapping sheet 10, the free portion 51 of the sealing tape 50 is attached to the first portion p1, and the fixed portion 52 of the sealing tape 50 is attached to the second portion p2. Note that the length in the lengthwise direction D1 of the second portion p2 can be 60% to 150% of the length in the lengthwise direction D1 of the first portion p1.

An end portion 51a on the free portion 51 side of the sealing tape 50 is not necessarily attached to the wrapping sheet 10. For example, as illustrated in FIG. 1, FIG. 3, and FIG. 4, the end portion 51a may be formed by folding the end portion on the free portion 51 side of the sealing tape 50 such that the outer surface (that does not have an adhesive layer) of the folded portion of the sealing tape 50 faces the wrapping sheet 10. Accordingly, the user can readily lift the free portion 51 of the sealing tape 50 by pinching the end portion 51a when opening the wrapped absorbent article 100.

The width of the sealing tape 50 is preferably 5 mm to 30 mm and more preferably 10 mm to 20 mm. Further, the entire length of the sealing tape 50 is preferably 10 mm to 50 mm and more preferably 15 mm to 40 mm.

### (Treatment on Outer Surface of Second Portion)

In the wrapping sheet 10 according to the present embodiment, the outer surface of the second portion p2 is treated. Treating the outer surface of the second portion p2 allows the bonding force between the second portion p2 and the sealing tape 50 to be smaller than the bonding force between the first portion p1 and the sealing tape 50. More specifically, the bonding force between the second portion p2 and the adhesive layer of the sealing tape 50 is smaller than the bonding force between the first portion p1 and the adhesive layer of the sealing tape 50. Therefore, the force required to peel the free portion 51 of the sealing tape 50 attached to the second portion p2 is smaller than the force required to peel the fixed portion 52 of the sealing tape 50 attached to the first portion p1. Accordingly, the free portion 51 can be readily peeled, and thus, an action to open the wrapped absorbent article 100 can be smoothly started.

In the conventional technique in which the surface state of the first portion p1 is the same as that of the second portion p2, a sealing tape 50 with low adhesiveness is sometimes used such that a free portion 51 of the sealing tape 50 can be readily peeled. However, in the conventional technique, if the entire sealing tape 50 has low adhesiveness, the adhesiveness of a fixed portion 52 of the sealing tape 50 would become low as well. Thus, before or while the wrapped absorbent article 100 is opened, the fixed portion 52 of the sealing tape 50 would be removed from the first region R1, and the original function of the sealing tape 50 to prevent the first region R1 from being opened would not be achieved. Conversely, in the present embodiment, the bonding force between the second portion p2 and the free portion 51 of the sealing tape 50 can be reduced without changing the configuration of the sealing tape 50. Accordingly, the free portion 51 can be readily peeled while maintaining the bonding force between the first region R1 and the fixed portion 52 of the sealing tape 50 to be high.

In the present embodiment, the surface of the second portion p2 is subjected to treatment for reducing the bonding force between the second portion p2 and the sealing tape 50, instead of subjecting the surface of the first portion p1 to treatment for increasing the bonding force between the first portion p1 and the sealing tape 50. The first portion p1 is a portion located in contact with the one end 11 of the wrapping sheet 10. Therefore, if misalignment occurs at the time of treatment of the wrapping sheet, there would be a possibility that only a part of the first portion p1, to which the sealing tape is to be attached, would be treated. In particular, the possibility would be high if the length of the first portion p1 is small. In such a case, even if the conditions of subsequent processes are changed (folding positions, tape attachment positions, and the like are changed), a desired wrapped absorbent article would not be obtained. Conversely, the second portion p2 is apart from the opposite end 12 of the second region R2. Therefore, even if misalignment occurs at the time of treatment of the wrapping sheet, a desired wrapped absorbent article can be obtained by making the length of a region to be treated greater than the length of the second portion p2 or by changing the conditions of subsequent processes.

The treatment for reducing the bonding force between the second portion p2 and the sealing tape 50 is not particularly limited. The treatment preferably includes forming a coating layer 35 having a low adhesive affinity on the second portion p2.

The coating layer 35 having a low adhesive affinity can be formed by, for example, applying a coating liquid to at least the second portion p2. The coating liquid includes, for example, a release agent component or a parting agent component, such as a silicone resin, a fluoropolymer, or rubber. Alternatively, the coating layer 35 having a low adhesive affinity can be formed by applying a thin sheet (release sheet), containing the above-described parting agent component in at least its surface, to the second portion p2.

Further, when the user replaces the used absorbent article with a new absorbent article, in most cases, the user removes a wrapping sheet from the new absorbent article first and wraps the used absorbent article in the wrapping sheet for disposal. At this time, after the user rolls the used absorbent article and wraps the used absorbent article in the wrapping sheet, the user can secure the used absorbent article by the sealing tape 50 that remains attached to the first region R1. More specifically, the used absorbent article is wrapped so that the first region R1 having the sealing tape 50 is located on the outermost side of the wrapping sheet 10. Then, the free portion 51 of the sealing tape 50 is attached to the surface of the wrapping sheet 10 beneath the free portion 51. Accordingly, the used absorbent article can remain rolled and wrapped in a compact manner. For the above reason, the free portion 51 of the sealing tape 50, which has been peeled once at the time of opening, is preferably re-usable (re-attachable).

In particular, if the wrapping sheet 10 is a fiber-containing sheet, fibers on the surface of the wrapping sheet 10 would tend to adhere to the adhesive layer of the sealing tape 50 when the sealing tape 50 is peeled to open the wrapped absorbent article. The fibers adhering to the adhesive layer of the peeled sealing tape 50 would decrease the adhesiveness of the adhesive layer. Conversely, according to the present embodiment, the coating layer 35 having a low adhesive affinity is formed on the second portion p2 to which the free portion 51 of the sealing tape 50 is attached, and at least a portion of the surface of the fiber-containing sheet is covered by the coating layer 35. Therefore, the adhesive layer of the free portion 51 of the sealing tape 50 does not directly contact at least a portion of the surface of the second portion p2. Accordingly, fibers do not or do not readily adhere to the free portion 51 of the sealing tape 50. Further, the adhesiveness of the free portion 51 of the sealing tape 50 after the wrapped absorbent article is opened (after the sealing tape 50 is peeled) can be improved. Therefore, when the used absorbent article is wrapped in the wrapping sheet 10 and secured by the sealing tape 50 for disposal, the sealing tape 50 can be more securely attached to the wrapping sheet 10, thus allowing the used absorbent article to remain wrapped in the wrapping sheet 10.

As illustrated in FIG. 1 through FIG. 3, the coating layer 35 having a low adhesive affinity may be continuously formed in a region (a low adhesive affinity region 30) over a predetermined length in the lengthwise direction D1 and over the entire width in the widthwise direction D2. However, the coating layer 35 having a low adhesive affinity may be discontinuously formed depending on the material of the wrapping sheet 10, the adhesiveness of the adhesive layer of the sealing tape 50, the configuration of the absorbent article, and the like. By discontinuously forming the coating layer 35, the material of the coating layer 35 can be reduced, and the wrapping sheet 10 can be thus manufactured at a lower cost. Further, other than the low adhesive affinity region 30, any coating layer having a low adhesive affinity is not preferably formed on the outer surface of the wrapping sheet 10. Accordingly, when the used absorbent article is rolled and wrapped in the wrapping sheet 10 for disposal, the sealing tape 50 can be securely attached to the outer surface of the wrapping sheet, thus allowing the used absorbent article to remain rolled and wrapped in a compact manner.

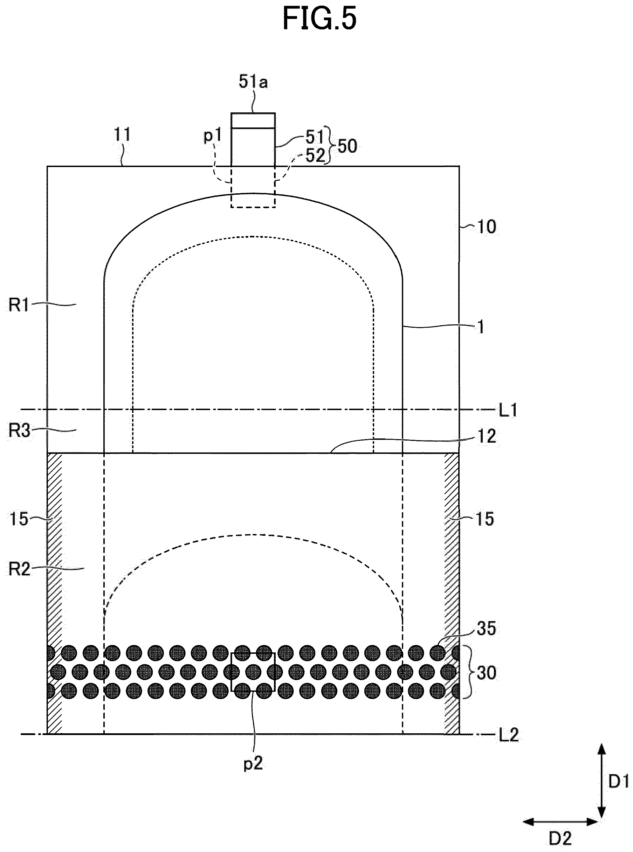

FIG. 5 illustrates an example in which the wrapping sheet 10 includes a discontinuous coating layer 35 having a plurality of portions. As illustrated, if the discontinuous coating layer 35 having the plurality of portions are formed, the portions of the coating layer 35 may be spaced apart from each other in the lengthwise direction D1 and/or in the widthwise direction D2.

In the example illustrated in FIG. 5, each of the portions of the discontinuous coating layer 35 has a circular shape in a plan view. However, the shape of each of the portions of the discontinuous coating layer 35 in a plan view is not limited to a circular shape, and may be a polygonal shape such as an elliptical shape, a triangle shape, or a square shape, or any other shape such as a heart shape, a star shape, or a droplet shape. Further, the coating layer 35 may be formed in a linear shape or a groove shape.

Further, the amount (basis weight) of the coating layer 35 applied to the second portion p2 per unit area can be made different depending on the position in the lengthwise direction D1. For example, with the absorbent article being wrapped, the amount of coating applied to the side of the coating layer 35 far from the one end 11 of the wrapping sheet 10 can be made larger than the amount of coating applied to the side closer to the one end 11. Accordingly, the bonding force between the sealing tape 50 and the wrapping sheet 10 can be reduced on the side closer to the end portion 51a of the sealing tape 50. Therefore, when the sealing tape 50 starts to be peeled, the sealing tape 50 can be readily peeled, thus facilitating smooth opening of the wrapped absorbent article.

Further, the area of the coating layer 35 formed on the second portion p2 per unit area can be made different depending on the position in the lengthwise direction D1. For example, with the absorbent article being wrapped, the area of the coating layer 35 per unit area can be larger on the side far from the one end 11 of the first portion p1 of the wrapping sheet 10 than the side closer to the one end 11. Accordingly, similar to the above-described example in which the amount of coating is different depending on the position in the lengthwise direction D1, the sealing tape 50 can be readily peeled when the sealing tape 50 starts to be peeled, thus facilitating smooth opening of the wrapped absorbent article.

The ratio of the total area of the coating layer 35 to the area of the second portion p2 in a plan view can be preferably 40% or more, and more preferably 60% or more. In order to reduce the bonding force between the second portion p2 and the sealing tape 50, the ratio of the total area of the coating layer 35 to the area of the second portion p2 in a plan view is preferably 100%, as illustrated in FIG. 1 and FIG. 3.

Note that the first portion p1 of the first region R1 can include a coating layer 35 having a low adhesive affinity, as long as the coating layer 35 does not reduce the bonding force between the fixed portion 52 of the sealing tape 50 and the first portion p1, and does not cause the fixed portion 52 to be removed from the first portion p1 before or when the wrapped absorbent article is opened. However, because the bonding force between the fixed portion 52 of the sealing tape 50 and the first portion p1 is preferably high, it is not preferable for the first portion p1 to include a coating layer 35 having a low adhesive affinity.

In the following, specific embodiments of the present invention will be appended.

### (Appendix 1)

According to an embodiment described in Appendix 1, a wrapping sheet for wrapping an absorbent article is provided. The wrapping sheet includes a first region and a second region. The first region includes one end in a first direction of the wrapping sheet and the second region includes an opposite end in the first direction. The wrapping sheet is configured to wrap the absorbent article by causing the first region to overlap with the outer surface of the second region, and a sealing tape is attached so as to extend from a first portion on the outer surface of the first region to a second portion on the outer surface of the second region. The outer surface of the second portion is subjected to treatment so as to reduce a bonding force between the second portion and the sealing tape as compared to a bonding force between the first portion and the sealing tape.

According to the embodiment described in Appendix 1, the second portion is subjected to treatment so as to reduce the bonding force between the second portion and the sealing tape as compared to the bonding force between the first portion and the sealing tape. That is, the bonding force between the wrapping sheet and the adhesive layer of the sealing tape is reduced in the second portion. Therefore, when the user opens the wrapped absorbent article while holding the free portion of the sealing tape, the free portion can be peeled from the second region with a smaller force. Further, according to the embodiment, without the need to change the sealing tape to a sealing tape having low adhesiveness, the free portion of the sealing tape can be readily peeled from the second region when the wrapped absorbent article is opened. Accordingly, the bonding force between the fixed portion of the sealing tape and the first portion of the wrapping sheet can be maintained to be high.

### (Appendix 2)

According to an embodiment described in Appendix 2, the treatment includes forming a coating layer having a low adhesive affinity.

According to the embodiment described in Appendix 2, a coating layer having a low adhesive affinity is formed on the outer surface of the second portion to which the sealing tape is attached.
Therefore, an affinity between the second portion and the adhesive provided on the wrapping sheet side of the sealing tape can be reduced. Accordingly, the bonding force between the free portion of the sealing tape and the wrapping sheet can be reduced. Therefore, when the user opens the wrapped absorbent article while holding the free portion of the sealing tape, the free portion can be peeled from the second region with a smaller force.

Further, according to the embodiment, without reducing the adhesiveness of the sealing tape, the free portion of the sealing tape can be readily peeled from the second region when the wrapped absorbent article is opened. Accordingly, the bonding force between the fixed portion of the sealing tape and the first portion of the wrapping sheet can be maintained to be high.

### (Appendix 3)

According to an embodiment described in Appendix 3, the coating layer is formed by applying a release agent or by applying a release sheet.

According to the embodiment described in Appendix 3, the coating layer having a low adhesive affinity can be formed in a simple manner.

### (Appendix 4)

According to an embodiment described in Appendix 4, with the absorbent article being wrapped, an area of the coating layer per unit area is larger on a side close to the one end than on a side far from the one end.

According to the embodiment described in Appendix 4, at a position where the user starts to peel the sealing tape when opening the wrapped absorbent article while holding the free portion of the sealing tape, an affinity between the adhesive of the sealing tape and the wrapping sheet is set to be lower and a bonding force therebetween is set to be smaller. Accordingly, a force required to start peeling the sealing tape can be small, and thus, the sealing tape can be smoothly peeled.

### (Appendix 5)

According to an embodiment described in Appendix 5, the wrapping sheet is a fiber-containing sheet.

According to the embodiment described in Appendix 5, sounds generated when the wrapped absorbent article is carried and opened can be reduced, and a soft texture can be provided.

### (Appendix 6)

According to an embodiment described in Appendix 6, the fiber-containing sheet is paper.

According to the embodiment described in Appendix 6, the environmental load at the time of disposal can be reduced, and also a unique texture can be provided.

### (Appendix 7)

According to an embodiment described in Appendix 7, a wrapped absorbent article includes the wrapping sheet according to any one of Appendixes 1 to 6, and an absorbent article.

According to the embodiment described in Appendix 7, the wrapped absorbent article having the same effect as that of any one of the embodiments of Appendixes 1 to 6 can be provided.

This application is based on and claims priority to Japanese Patent Application No. 2020-031876, filed on February 27, 2020, the entire contents of which are incorporated herein by reference.

### DESCRIPTION OF THE REFERENCE NUMERALS

1 absorbent article
10 wrapping sheet
11 one end in lengthwise direction
12 opposite end in lengthwise direction
15 seal portion
30 low adhesive affinity region
35 coating layer
50 sealing tape
51 free portion of sealing tape
51a end portion on free portion side
52 fixed portion of sealing tape
100 wrapped absorbent article
D1 free portion of wrapping sheet
D2 second direction of wrapping sheet
L1 first folding line
L2 second folding line
p1 first portion
p2 second portion
R1 first region
R2 second region
R3 third region

## Claims

1. A wrapping sheet for wrapping an absorbent article, the wrapping sheet comprising:
a first region and a second region, the first region including one end in a first direction of the wrapping sheet and the second region including an opposite end in the first direction,
wherein the wrapping sheet is configured to wrap the absorbent article by causing the first region to overlap with an outer surface of the second region, and a sealing tape is attached so as to extend from a first portion on an outer surface of the first region to a second portion on the outer surface of the second region, and
wherein an outer surface of the second portion is subjected to treatment so as to reduce a bonding force between the second portion and the sealing tape as compared to a bonding force between the first portion and the sealing tape.

2. The wrapping sheet according to claim 1,
wherein the treatment includes forming a coating layer having a low adhesive affinity.

3. The wrapping sheet according to claim 2,
wherein the coating layer is formed by applying a release agent or by applying a release sheet.

4. The wrapping sheet according to claim 2 or 3, wherein, with the absorbent article being wrapped, an area of the coating layer per unit area is larger on a side far from the one end than on a side closer to the one end.

5. The wrapping sheet according to any one of claims 1 to 4, wherein the wrapping sheet is a fiber-containing sheet.

6. The wrapping sheet according to claim 5,
wherein the fiber-containing sheet is paper.

7. A wrapped absorbent article, comprising the wrapping sheet according to any one of claims 1 to 6, and an absorbent article.
